(19) Europäisches Patentamt / European Patent Office / Office européen des brevets

(11) **EP 2 853 917 B1**

(12) **EUROPEAN PATENT SPECIFICATION**

(45) Date of publication and mention
of the grant of the patent:
**15.01.2020 Bulletin 2020/03**

(51) Int Cl.:
*G01S 15/89* (2006.01)       *A61B 8/08* (2006.01)
*A61B 8/00* (2006.01)       *A61B 5/00* (2006.01)
*G01S 7/52* (2006.01)

(21) Application number: **14181957.3**

(22) Date of filing: **22.08.2014**

(54) **Photoacoustic and ultrasound echo imaging apparatus and method for controlling same**

Photoakustische und Ultraschallechoabbildungsvorrichtung und Verfahren zur Steuerung davon

Appareil d'acquisition d'images photoacoustiques et ultrasonores et son procédé de commande

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.09.2013 JP 2013199836**

(43) Date of publication of application:
**01.04.2015 Bulletin 2015/14**

(73) Proprietor: **Canon Kabushiki Kaisha
Tokyo 146-8501 (JP)**

(72) Inventor: **Tateyama, Jiro
Tokyo,, Tokyo 146-8501 (JP)**

(74) Representative: **TBK
Bavariaring 4-6
80336 München (DE)**

(56) References cited:
EP-A1- 1 561 424        WO-A1-2011/074656
WO-A1-2013/065249        WO-A2-2010/137451

**Description**

BACKGROUND OF THE INVENTION

Field of the Invention

The present invention relates to an object information acquiring apparatus and a method for controlling the same.

Description of the Related Art

Conventionally, in an ultrasound diagnostic apparatus used for medical image diagnosis, a probe including a vibrating element having the function of transmitting/receiving an ultrasound wave has been used. When an ultrasound beam formed by synthesizing ultrasound waves is transmitted from the probe toward an object, the ultrasound beam is reflected by a region (i.e., the boundary between tissues) in the object where an acoustic impedance changes. Through the reception of the refection echo thereof by the probe and the image reconstruction by an information processor on the basis of the intensity thereof, two-dimensional image data (tomographic slice image) showing the structural distribution information of living tissues can be acquired.

According to the technique in Japanese Patent Application Laid-open No. 2010-269046, the surface of an object is mechanically scanned in X- and Y-directions using a one-dimensional array (1D array) of probes to allow continuous tomographic slice images to be obtained. As a result, the three-dimensional image data of a wide region under examination can be generated.

On the other hand, in Srirang Manohar, et al., "The Twente photoacoustic mammoscope: system overview and performance", Physics in Medicine and Biology, 50 (2005), 2543-2557, a living-body-information imaging apparatus for breast cancer examination is described to which a photoacoustic tomographic (PAT) technique is applied.

The PAT is a technique which visualizes information related to optical characteristic values in an object using a photoacoustic wave generated by a photoacoustic effect from the living tissue that has absorbed the energy of light propagated/diffused in the object. In practicing the PAT, the photoacoustic wave is detected at each of a plurality of places surrounding the object and the obtained signal is subjected to mathematical analysis processing.

The photoacoustic effect is a phenomenon in which, when an object is illuminated with pulsed light, a photoacoustic wave is generated through volume expansion in a region with a high absorption coefficient in the object.

In the PAT, the functional distribution information of living tissues showing the presence/absence of a specified component or a change therein, such as an initial acoustic pressure distribution or absorbed optical energy density distribution resulting from light illumination, can be acquired.

Additionally, in Japanese Patent Application Laid-open No. 2010-269046, a configuration is also described which performs mechanical scanning of the surface of the object in the X- and Y-directions using an integrated probe including a two-dimensional array (2D array) of probes for receiving the photoacoustic wave and the one-dimensional array of probes for transmitting/receiving an ultrasound wave.

Patent Literature 1: Japanese Patent Application Laid-open No. 2010-269046

Further prior art is known from documents WO 2011/074656 A1, EP 1 561 424 A1 and WO 2013/065249 A1.

Non Patent Literature 1: Srirang Manohar, et al., "The Twente photoacoustic mammoscope: system overview and performance", Physics in Medicine and Biology, 50 (2005), 2543-2557

SUMMARY OF THE INVENTION

However, when three-dimensional image data is generated through mechanical scanning using an integrated probe including two types of receiving functions as in Japanese Patent Application Laid-open No. 2010-269046, image data derived from both of an ultrasound echo and a photoacoustic wave cannot be recognized until full scanning with the probe is completed. The reason for this will be described below.

When image reconstruction based on the photoacoustic wave is to be performed, reception data from the entire imaging target region is required. Accordingly, during the scanning of the imaging target region, it is impossible to sequentially generate images from one scanned place after another and display the generated images in real time. Therefore, the three-dimensional image data has been generated conventionally by performing image reconstruction processing after the full scanning with the probe is completed. Note that, depending on cases, the imaging target region may indicate the entire object or one of partial regions into which the object has been divided.

On the other hand, with regard to ultrasound image reconstruction, real-time image generation is normally possible. However, in the apparatus in Japanese Patent Application Laid-open No. 2010-269046, an ultrasound image has been generated so as to be mainly displayed in superimposition on the photoacoustic image. Accordingly, the apparatus in P Japanese Patent Application Laid-open No. 2010-269046 is not provided with the function of displaying the ultrasound image before the full scanning with the probe is completed.

The present invention has been achieved in view of the foregoing problem. The present invention is developed to allow, in an apparatus which generates the image data of a target region in an object using a photoacoustic wave and an ultrasound echo, the image data to be recognized without waiting for the completion of scanning of the entire target region using a probe.

The present invention in its first aspect provides an object information acquiring apparatus as specified in claims 1 to 8.

The present invention in its second aspect provides a method for controlling an object information acquiring apparatus as specified in claims 9 to 16.

According to the present invention, in the apparatus which generates the image data of the target region in the object using the photoacoustic wave and the ultrasound echo, the image data can be recognized without waiting for the completion of scanning of the entire target region using the probe.

Further features of the present invention will become apparent from the following description of a comparative example and an exemplary embodiment with reference to the attached drawings.

BRIEF DESCRIPTION OF THE DRAWINGS

FIG. 1 is a view showing an overall configuration of the present invention;
FIG. 2 is a view schematically showing an apparatus according to a comparative example;
FIG. 3 is a view showing a mechanical operation of a probe;
FIG. 4 is a view showing the procedure of scanning with an ultrasound probe;
FIGS. 5A to 5C are 3-plane views each showing an object;
FIG. 6A shows views of C-mode images at a depth Z;
FIG. 6B shows other views of C-mode images at a depth Z;
FIGS. 7A and 7B are views in each of which B-mode images at a scanning position X are continuously displayed;
FIGS. 8A and 8B are views in each of which a camera image and a C-mode image are simultaneously displayed;
FIGS. 9A and 9B are views each showing an image after the adjustment of a ROI start point; and
FIGS. 10A and 10B are views each showing an image after the adjustment of a ROI end point.

DESCRIPTION OF THE EMBODIMENTS

Referring now to the drawings, preferred embodiments of the present invention will be described below. However, the dimensions, materials, and shapes of components described below, relative positioning thereof, and the like are to be appropriately changed in accordance with a configuration of an apparatus to which the invention is applied and various conditions and are not intended to limit the scope of the invention to the following description.

In the present invention, an acoustic wave includes an elastic wave or a compressional wave referred to as a sound wave, an ultrasound wave, a photoacoustic wave, or a photo-ultrasound wave. An object information acquiring apparatus of the present invention serves as each of a photoacoustic tomographic apparatus and an ultrasound apparatus. The former apparatus illuminates an object with light (an electromagnetic wave) and receives a photoacoustic wave generated by a photoacoustic effect in an object to acquire the characteristic information on the interior of the object. The latter apparatus transmits an ultrasound wave to the object and receives the ultrasound wave (reflection echo) reflected in the object to acquire the characteristic information on the interior of the object.

The characteristic information acquired by photoacoustic tomography (PAT) is object information reflecting the initial acoustic pressure of an acoustic wave generated by light illumination, the density of absorbed optical energy and an absorption coefficient each derived from the initial acoustic pressure, the concentrations of substances forming tissues, and the like. It can be said that, since the substances forming the tissues reflect functions, a photoacoustic characteristic distribution represents the functional distribution information of the object.

Examples of the concentrations of the substances include the degree of oxygen saturation, an oxyhemoglobin concentration, and a deoxyhemogrobin concentration. The generated characteristic information may also be stored and used as numerical value data, distribution information at each location in the object, or image data for displaying an image.

The characteristic information acquired by the transmission/reception of an ultrasound wave is object information reflecting a segment in the object in which an acoustic impedance changes, i.e., the boundary position between regions having different acoustic impedances. Since the acoustic impedance difference reflects the structures of tissues, it can be said that an acoustic impedance characteristic distribution represents the structural distribution information of the object.

Referring now to the drawings, the present invention will be described below in detail. Note that like components are denoted by like reference numerals in principle and a description thereof may be omitted. The present invention can be embodied as an object information acquiring apparatus, an operating method therefor, or a control method therefor. The present invention can also be embodied as a program which causes an information processor or the like to implement the control method.

(Image Reconstruction Technique)

Theoretically, in the PAT, if a time change in acoustic wave can be measured using an ideal acoustic detector at different points over the surface of a confined space (particularly a spherical surface) surrounding the entire object, an initial acoustic pressure distribution resulting from light illumination can completely be visualized. The ideal acoustic detector indicates a wideband point detector.

It is also mathematically known that, even when measurement is not performed at a confined space, if an acoustic wave can be measured at the surface of a cylindrical space surrounding the object or at the surface of a flat-plate-shaped space facing the object, the initial acoustic pressure distribution can substantially be reproduced.

The following equation (1) is a partial differential equation forming the basis of the PAT and referred to as "photoacoustic wave equation". By solving the equation, acoustic wave propagation from the initial acoustic pressure distribution can be described and where and how the acoustic wave can be detected can theoretically be determined.
[Math. 1]

$$\left(\nabla^2 - \frac{1}{c^2}\frac{\partial^2}{\partial t^2}\right)p(r,t) = -p_0(r)\frac{\partial\delta(t)}{\partial t} \qquad (1)$$

where r is a location, t is a time, $p(r, t)$ is a time change in acoustic pressure, $p_0(r)$ is an initial acoustic pressure distribution, c is an acoustic velocity, and $\delta(t)$ is a delta function representing the shape of a light pulse.

On the other hand, PAT image reconstruction is the derivation of the initial acoustic pressure distribution $p_0(r)$ from an acoustic pressure $p_d(r_d, t)$ obtained at a detection point, which is mathematically referred to as an inverse problem.

A description will be given below of a universal back projection (UBP) method which is used typically in a PAT image reconstruction method. By analyzing the photoacoustic wave equation of the equation (1) over a frequency space, the inverse problem for determining $p_0(r)$ can precisely be solved. A representation of the result thereof over a time space corresponds to a UBP. Eventually, the following equation (2) is derived.
[Math. 2]

$$p_0(r) = -\frac{2}{\Omega_0}\nabla\cdot\int_{S_0}\hat{n}_0^s dS_0\left[\frac{p_0(r_0,t)}{t}\right]_{t=|r-r_0|} \qquad (2)$$

where $\Omega_0$ is the solid angle of an overall measurement area $S_0$ with respect to an arbitrary reconstruction voxel (or focal point).

The equation is further deformed to be easily understandable, resulting in an equation (3).
[Math. 3]

$$p_0(r) = \int_{\Omega_0} b(r_0, t = |r - r_0|)\frac{d\Omega_0}{\Omega_0} \qquad (3)$$

where $b(r_0, t)$ is projection data, as shown in the equation (4), and $d\Omega_0$ is the solid angle of a detector $dS_0$ with respect to an arbitrary monitoring point P, as shown in the equation (5).

By subjecting the projection data to back projection in accordance with the integration in the equation (3), the initial acoustic pressure distribution $p_0(r)$ can be obtained.
[Math. 4]

$$b(r_0,t) = 2\,p(r_0,t) - 2t\frac{\partial p(r_0,t)}{\partial t} \qquad (4)$$

$$d\Omega_0 = \frac{dS_0}{|r - r_0|^2}\cos\theta \qquad (5)$$

In the foregoing equations, $\theta$ is the angle formed between the detector and the arbitrary monitoring point P. When the distance between an acoustic source and a measurement position is sufficiently large (acoustic far-field approximation) compared to the size of the acoustic source, an equation (6) is provided.
[Math. 5]

$$p(r_0,t) << t\frac{\partial p(r_0,t)}{\partial t} \qquad (6)$$

On the other hand, b(r0, t) is given by an equation (7).
[Math. 6]

$$b(r_0,t) = -2t\frac{\partial p(r_0,t)}{\partial t} \qquad (7)$$

Thus, in the PAT image reconstruction, by subjecting the detection signal $p(r_0, t)$ obtained by the detector to temporal differentiation, the projection data $b(r_0, t)$ can be obtained. It is known that, by subjecting the projection data $b(r_0, t)$ to back projection in accordance with the equation (3), the initial acoustic pressure distribution $p_0(r)$ can be obtained.
Thus, in the image reconstruction based on the PAT, the characteristic information on the interior of the object such as a living body can be imaged. The characteristic information includes the generation source distribution of the acoustic wave resulting from light illumination, an initial acoustic pressure distribution in the living body, an absorbed light energy density distribution derived therefrom, and the concentration distributions of the substances forming living tissues which can be obtained from the foregoing information items. Such characteristic information can be used for the purpose of, e.g., diagnosing a malignant tumor, a blood vessel disease, or the like or following up chemotherapy.

< Comparative example >

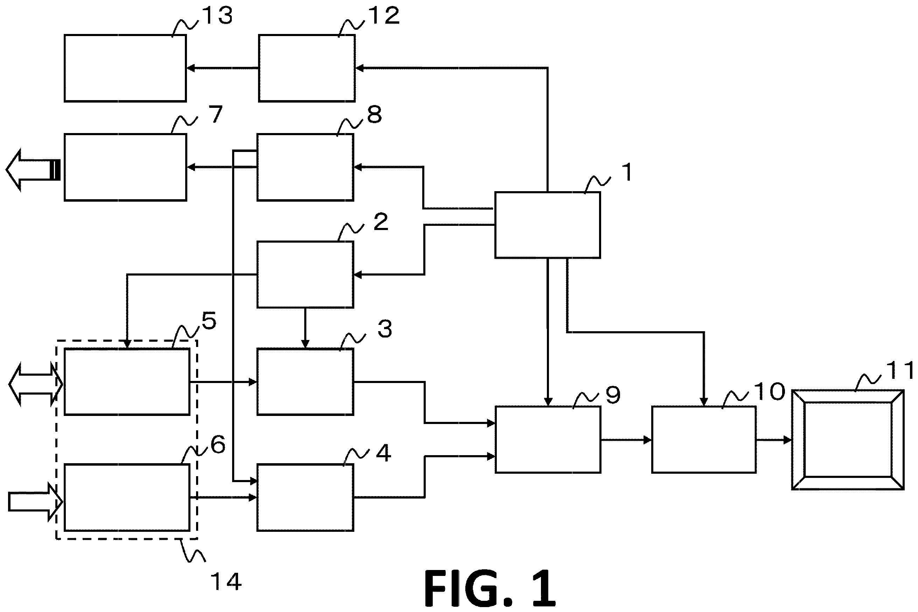

FIG. 1 is an overall configurational view of an object information acquiring apparatus most clearly representing the characteristic feature thereof. When an object is a living body, the apparatus may also be referred to as a living-body-information imaging apparatus.

(Apparatus Configuration and Operation)

A CPU 1 is responsible for the main control of the apparatus. An ultrasound wave transmission unit 2 drives an ultrasound probe to transmit an ultrasound beam. An ultrasound wave reception unit 3 retrieves the reception signal detected by the ultrasound probe to form a beam. A photoacoustic wave reception unit 4 retrieves the reception signal detected by a photoacoustic probe.
A 1D array of probes 5 generate an ultrasound wave and detects a reflection echo. A 2D array of probes 6 are used to detect the signal of a photoacoustic wave. A probe 14 is an integrated mechanism including the ultrasound probes 5 and the photoacoustic probes 6.
A light illumination unit 7 illuminates the object with light. A light source unit 8 controls the light illumination unit. An image processing unit 9 calculates image data using reception signals from the photoacoustic wave and the ultrasound wave. A display control unit 10 controls scan conversion of an image and superimposed display thereof. A display 11 displays image data. A scanning control unit 12 performs X-Y scanning movement of the integrated probe 14 to an arbitrary position. The scanning unit 13 performs mechanical scanning movement of the probe.

A basic operation for imaging based on the transmission/reception of an ultrasound wave will be described. When the ultrasound probe 5 is pressed against the object to transmit an ultrasound wave, the ultrasound wave travels in the object in an extremely short period of time to become a reflection echo at a boundary providing an acoustic impedance difference. The acoustic impedance difference means that different media are in contact. The probe detects the reflection echo.

Then, the image processing unit calculates a distance from the time between the transmission of the ultrasound wave and the return of the reflection echo to image tissues in the object. In this manner, "structural image" (structural distribution information)" representing the substance distributions of living tissues can be imaged.

The scanning control unit corresponds to the scanner of the present invention. The probe corresponds to the receiver of the present invention. The image processing unit corresponds to the processor of the present invention. The display corresponds to the display of the present invention.

A basic operation for image reconstruction based on the PAT will be described. First, the light illumination unit 7 driven by the light source unit 8 illuminates the object with pulsed light. Then, the probe 6 receives (detects) the photoacoustic wave generated through the absorption of the energy of the pulsed light propagated/diffused in the object by living tissues . By subjecting the resulting reception signal to reconstruction processing in the image processing unit, an optical characteristic distribution in the object, particularly an absorbed optical energy density distribution can be acquired. In this manner, a "functional image (functional distribution information) " representing the substance distributions of the living tissues can be imaged.

(About Probe Scanning)

In the PAT image reconstruction, as the region which receives the photoacoustic signal is larger in size, a reception aperture is larger in size, and this allows the resolution of a PAT image to be increased. However, the use of a large-area multi-element probe for the PAT significantly increases the number of channels of the reception unit for performing simultaneous parallel reception, leading to increases in the cost and size of the apparatus. In preventing this, probe-scanning-type PAT is effective. In addition, by integrating signals while performing scanning, an SN ratio can also be improved.

However, when a PAT apparatus has a probe-scanning-type configuration, it is necessary to store the signals acquired during scanning and perform image reconstruction using the stored reception signals (integrated signals) after the completion of the scanning. As a result, the configuration cannot obtain a PAT image before the scanning of at least an entire target region for the image reconstruction is completed. That is, when an image is reconstructed on the basis of the data of the entire region of the object, it is necessary to wait for the completion of full scanning. Even when reconstruction is performed on the basis of each one of partial regions such as stripes of blocks into which the object has been divided, it is necessary to wait for the completion of scanning in the partial region.

Here, even in such a scanning-type apparatus as using a probe having a common element serving as each of an ultrasound element and a photoacoustic element or an integrated probe including an ultrasound element and a photoacoustic element, a PAT image cannot be obtained until scanning is completed. As a result, a captured image is not displayed in real time and whether or not image sensing is correctly proceeding may not be able to be determined. In addition, the configuration may be such that a PAT image and an ultrasound image each partially produced are joined together.

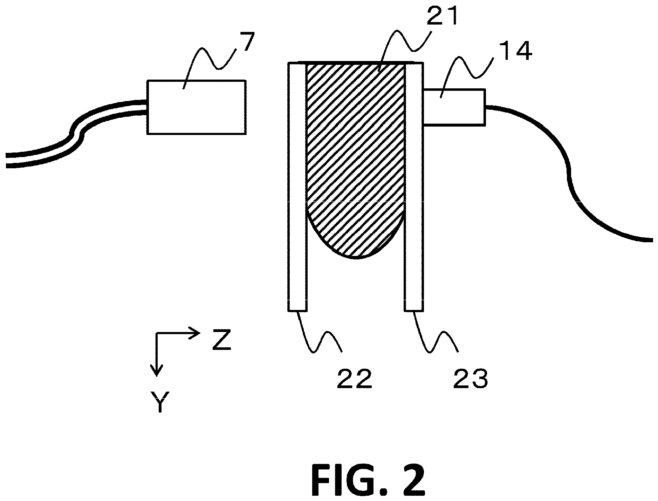

FIG. 2 is a partial schematic diagram of the object information acquiring apparatus. In the apparatus of the comparative example, when a breast of a person under examination is to be measured as an object, the person under examination is placed in a prone position and an object 21 is held between two plates (a pressing plate 22 and a holding plate 23). Since the distance between the pressing plate and the holding plate is adjustable, the intensity of the pressure under which the object is held and the thickness (thinness) of the object can be controlled.

The probe 14 receives the ultrasound wave and the photoacoustic wave each generated from the object via the holding plate holding the object. Between the probe and the holding plate or between the holding plate and the object, an acoustic matching material may also be placed. The probe is capable of mechanical scanning movement in X- and Y-directions along the surface of the holding plate.

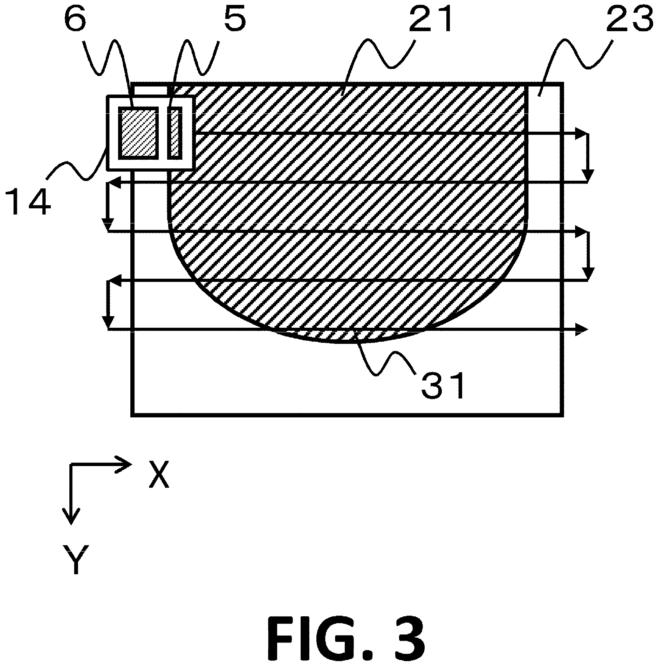

FIG. 3 is a view showing the mechanical scanning movement of the probe 14. As described above, the probe is the integrated probe including the 1D array of ultrasound probes 5 and the 2D array of photoacoustic probes 6. The probe moves over the object 21 via the holding plate 23 along a movement path 31.

First, the scanning control unit 12 rightwardly moves the probe in a horizontal direction (X-direction) along the surface of the holding plate. When the probe reaches the right end portion thereof, the scanning control unit 12 changes the direction of the movement thereof to a downward perpendicular direction (Y-direction). When the probe has moved over a predetermined distance in the Y-direction, the scanning control unit 12 leftwardly moves the probe in the horizontal direction again. By repeating such mechanical scanning movement of the probe, the scanning control unit allows the entire region under examination to be measured.

Here, a region formed by one scanning operation in the X-direction is referred to as a stripe. The apparatus measures the entire region by dividing the object into a plurality of the stripes. At the time of image reconstruction, the image reconstruction may be performed on the basis of each one of the stripes, or a plurality of or all the stripes may collectively be used as a unit for the image reconstruction. Alternatively, the partial regions may also be set without being limited to the stripes.

In this comparative example, the X-direction in which each of the stripes extends can be referred to as a main scanning direction and the Y-direction in which the probe moves between the stripes can be referred to as a subordinate scanning direction. The individual stripes may also overlap each other in the Y-direction.

Note that the timings for light illumination and photoacoustic wave acquisition in the mechanical scanning movement are arbitrary. For example, a method (step-and-repeat method) which intermittently moves the probe and performs measurement when the probe stops may also be used. Alternatively, a method which performs measurement while continuously moving the probe may also be used. In either of the methods, by performing an arithmetic operation in accordance with the position of the probe and measurement timing, the inside of the object can be reconstructed. This allows repetitive images to be acquired at different positions on the movement path 31.

(Generation and Display of Image Data)

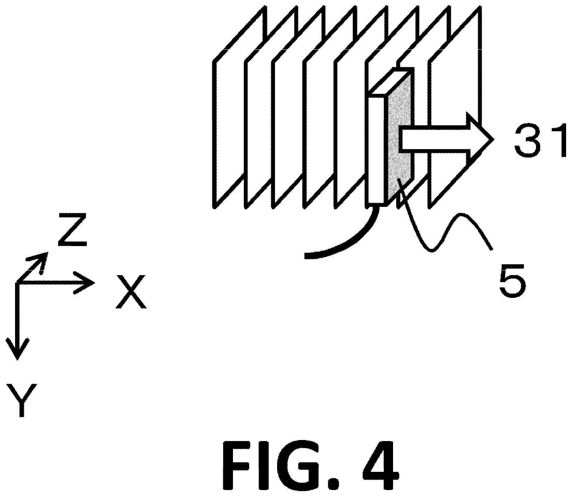

FIG. 4 shows the procedure of scanning when two-dimensional tomographic slice images serving as ultrasound images are acquired, while the probe moves along the movement path 31. The timing for outputting the tomographic slice image is as follows. When the probe is intermittently moved, the tomographic slice images are output at the stopping of the probe while, when the probe is continuously moved, the tomographic slice images are output at intervals of a given period. By arranging the acquired tomographic slice images, a three-dimensional ultrasound image of the entire region under examination can be constructed.

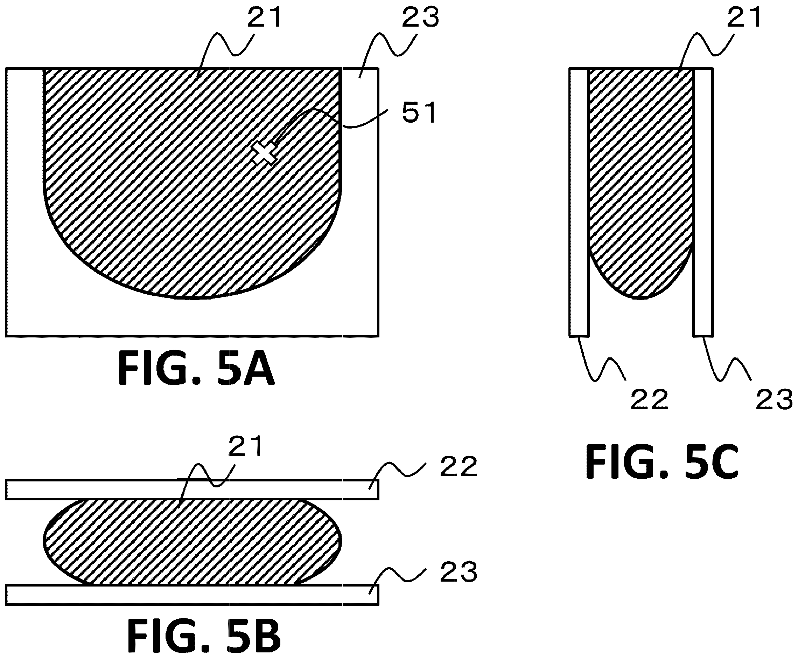

FIGS. 5A to 5C are 3-plane views each showing the shape of the object when the object is held between the holding plate and the pressing plate. FIGS. 5A to 5C show the respective images of the object captured from three directions using an imaging unit such as a camera. At the time of diagnosis by a medical personnel or the like, a PAT image or an ultrasound image may also be displayed in superimposition on each of the 3-plane views. Note that a marking 51 is displayed to specify the location of a lesion in response to a designation by an operator.

When the three-dimensional image data resulting from image reconstruction based on the PAT is present, the display control unit extracts 3-plane slice images using a volume rendering function to allow the 3-plane slice images specifying an arbitrary location (X-, Y-, and Z coordinates) in the object to be displayed.

On the other hand, ultrasound three-dimensional image data can be sequentially imaged in three dimensions by arranging the acquired tomographic slice images even while the probe is moved for scanning. That is, 3-plane slice images which are a C-mode image in an X-Y plane along the holding plate, a tomographic slice image (B-mode image) in a Y-Z plane along the arrangement of the probe elements, and an elevation image corresponding to an X-Z plane can be extracted.

At this time, in the image reconstruction based on the PAT, the reception data of the entire object (or of the entire partial region as an image reconstruction target when the reconstruction is performed on the basis of each one of the partial regions into which the object has been divided) is necessary. Accordingly, reconstruction processing is performed after the completion of scanning of the entire region to generate image data.

On the other hand, in the ultrasound image reconstruction, the probe is moved for scanning to sequentially obtain the tomographic slice images of the other regions. As a result, the image data of the regions scanned with the probe is sequentially generated. That is, the PAT image is displayed by performing image reconstruction after the completion of full scanning, while the ultrasound image can be displayed in real time while scanning is performed.

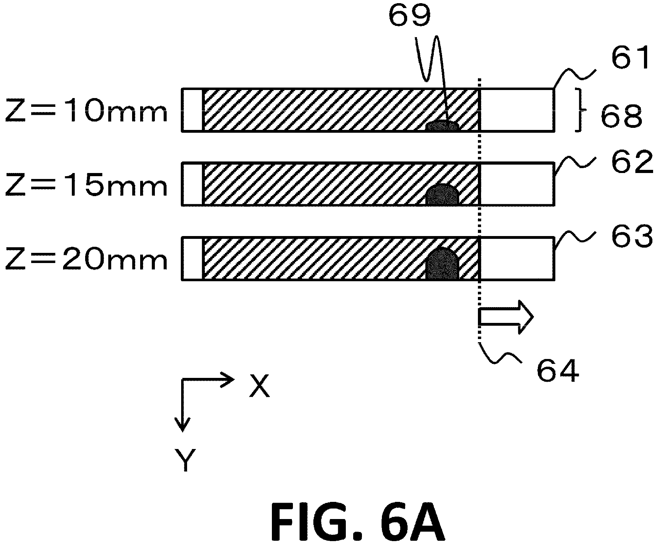

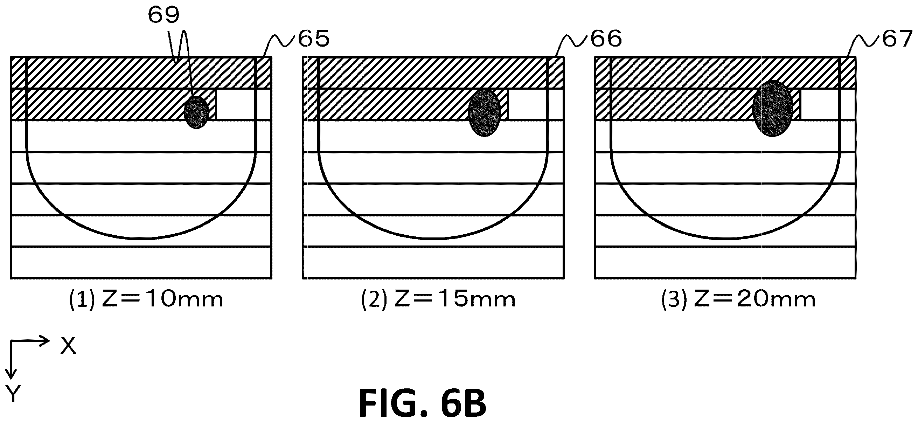

FIGS. 6A and 6B show display examples when ultrasound images are displayed in real time with the scanning using the probe. In each of the drawings, a plurality of C-mode images at a depth Z are displayed. In these examples, with the scanning movement of the probe in the X-direction, the C-mode images at the depths Z of 10 mm, 15 mm, and 20 mm are displayed on the display. That is, even during the period during which data for generating photoacoustic images is acquired, the C-mode images are displayed.

FIG. 6A shows C-mode images 61, 62, and 63 corresponding to the respective depths of 10 mm, 15 mm, and 20 mm. As the probe moves to a scanning position 64 in the X-direction, the images are sequentially generated and displayed on the display. When the probe moves in the Y-direction and the stripes are changed, the images are cleared and the next stripe is newly displayed.

FIG. 6B shows C-mode images 65, 66, and 67 corresponding to the respective depths of 10 mm, 15 mm, and 20 mm. In the method of FIG. 6B, by displaying the previous images even when the current stripe is changed to the next stripe in the Y-direction, an image of the entire object is finally displayed.

The display method is appropriate for the case where structural information when a lesion 69 has extended in the depth direction Z is to be recognized. The method is most appropriate for the case where a comparison is made to the shape of the lesion recognized with another modality.

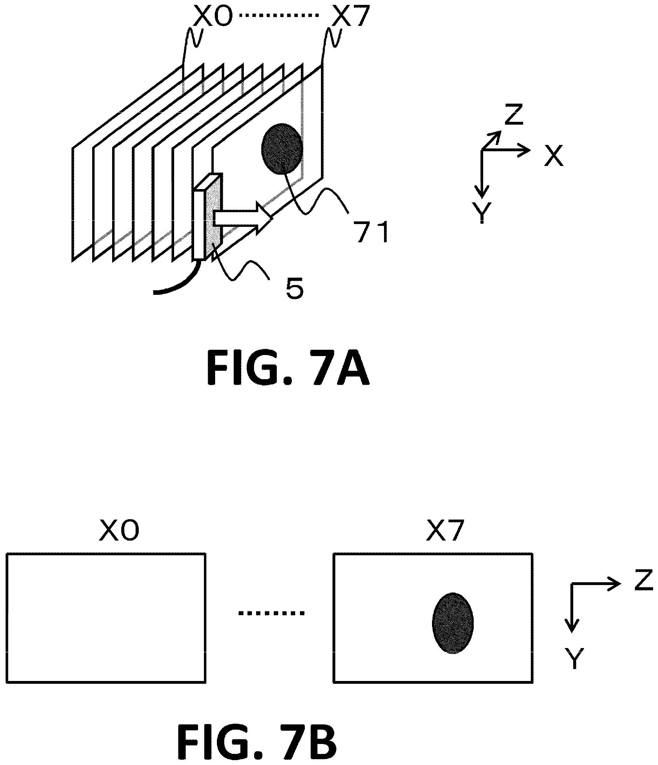

FIGS. 7A and 7B show, as an example when an ultrasound image is displayed in real time with the scanning using the probe, views when the B-mode images at a scanning position X are continuously displayed even during the period during which scanning is performed.

FIG. 7A is a view showing tomographic slice images X0 to X7 corresponding to the probe scanning positions in the X-direction. FIG. 7B is a view continuously displaying the tomographic slice images X0 to X7 on the display. At this time, a method which continuously displays the individual images at the same position or different positions may be used or a method which selectively displays the image at the designated position in the X-direction may also be used. Alternatively, the images in accordance with both of the methods may also be simultaneously displayed.

Here, a mass 71 shows a lesion. When a comparison is made between the displayed tomographic slice images, the mass 71 is not observed in X0 but is observed in X7. This shows that the ultrasound image changes depending on the probe scanning position.

The display method is appropriate for the case where structural information when the lesion has extended in the scanning direction X is to be recognized. The method is appropriate when a real-time image corresponding to the probe scanning position is to be recognized.

As has been shown in the comparative example using the various examples of image display, the present invention allows the ultrasound image data to be sequentially displayed while moving the probe for scanning in the X- and Y-directions relative to the object. As a result, in the object information acquiring apparatus using both of the ultrasound image and the photoacoustic image, the effect of allowing an image sensing state (apparatus operating state or the progress of image sensing) to be recognized in real time without waiting for the completion of the photoacoustic tomography can be obtained. As a result, the user can check up a required operation while recognizing the image as necessary.

<Embodiment>

In the present embodiment, a method of applying the present invention to an object information acquiring apparatus which sets a region of interest (ROI) in an object and the effect thereof will be described. The apparatus of the present embodiment has an input unit which receives a designation input from the user and sets the ROI in the surface of the target.

When the image data of the object 21 is acquired on the basis of the PAT, it is preferable to preliminarily estimate the X- and Y-positions of the lesion 71 from the result of diagnosis using another modality (MRI, X-ray mammography, or an ultrasound wave), set the periphery of the lesion as the ROI, and perform measurement. By setting the ROI, it is possible to reduce a measurement time when there is no need to measure the entire object and generate detailed image data aimed at only a required region.

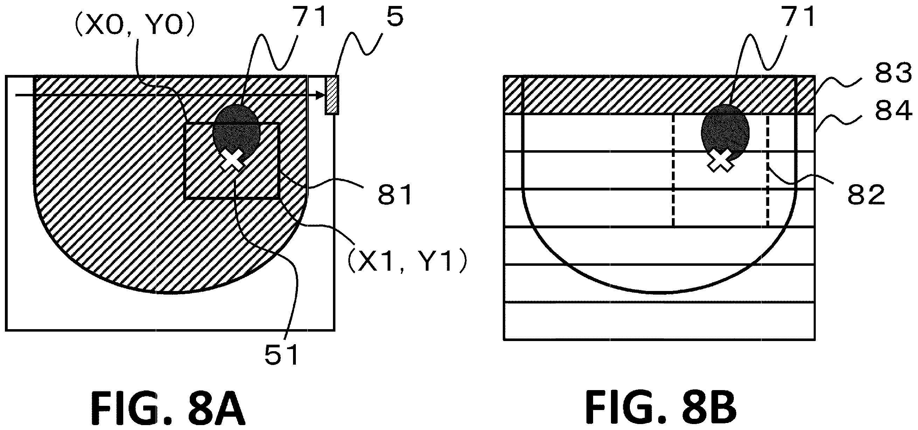

FIGS. 8A and 8B show the images displayed on the display in the present embodiment. FIG. 8A is a camera image from which the probe scanning position can be recognized. FIG. 8B is a C-mode image generated in real time. By simultaneously displaying these images, it is possible to recognize the probe scanning position and the relative position of the lesion on a screen.

In the camera image of FIG. 8A, a range 81 shows the initially set range of the region of interest (ROI). Through the setting, PAT image data is acquired from the range defined by a start point (X0, Y0) and an end point (X1 and Y1).

Note that the position and size of the ROI can arbitrarily be changed. Also, the shape of the ROI is not limited to a rectangle. It is possible to recognize the set position while viewing the position of the marking in an object image in the camera image. The position of the ROI can be determined by an arbitrary method such as the specification of coordinates or a specification with a touch pen by the user.

In the C-mode image of FIG. 8B, a region 82 shows the range of the ROI which is set in units of stripes. The region 82 is set in units of stripes such that the set range 81 of the ROI in the camera image is sufficiently included therein as necessary. The region 82 can be automatically determined on the basis of information related to, e.g., the sizes of the region 81 and the probe and a scanning path.

When the ROI is set, a marking 51 is formed on the surface of the object to specify the position of the lesion 71 such that the center of the ROI is aligned with the position while the camera image is viewed. However, when the object is held between the holding plate and the pressing plate, the shape of the object may be changed, whereby the position of the marking is deviated from the real lesion.

However, the image reconstructed on the basis of the PAT cannot be recognized until the scanning of the entire imaging target region (which is the ROI in this case) is terminated. Consequently, when the position is shifted, a problem occurs in that, e.g., a part of the reconstructed image becomes a waste or the portion needed for diagnosis cannot be imaged. Note that the setting of the region of interest may also be performed automatically on the basis of the marking position.

If the apparatus shown in the comparative example is used, such a problem can be solved. That is, in this apparatus, the C-mode images obtained by the transmission/reception of an ultrasound wave are sequentially displayed. By recognizing the precise position of the lesion using such images in real time, whether or not the set range of the ROI is proper can be determined as needed. This allows the user to adjust the set range in real time.

FIG. 8B shows the C-mode image in units of stripes. At the stage at which the C-mode image of a stripe 83 located at an upper position is displayed, a part of the lesion 71 is displayed. Accordingly, it can be recognized that the set range 82 of the ROI is out of the lesion 71. In other words, by observing the stripe 83, it becomes clear that the set range 81 of the ROI is out of the lesion 71. Therefore, it is necessary to change the set position of the ROI before the data of a next stripe 84 is acquired and acquire the data of the stripe 83 again.

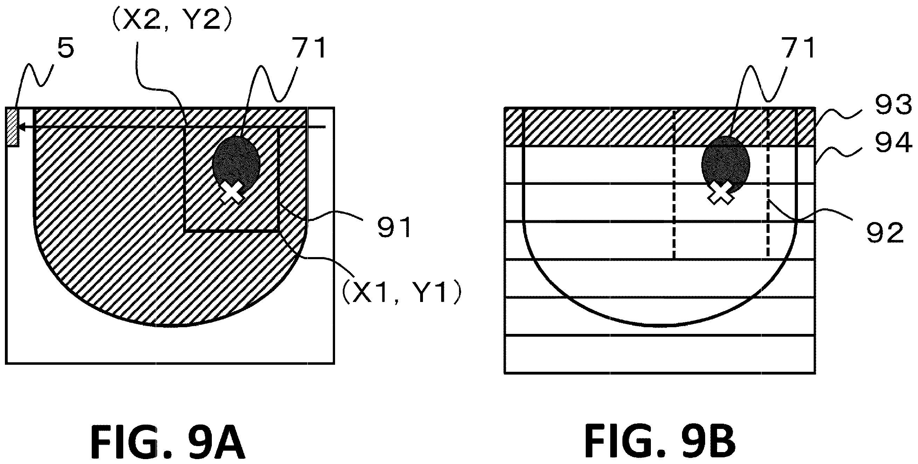

FIGS. 9A and 9B show the state after the set range has been adjusted with respect to the start point of the ROI. FIG. 9A is a cameral image and FIG. 9B is a C-mode image. The range 91 shows a set range after ROI adjustment in the camera image (FIG. 9A). By the setting, the start point of the ROI is changed from (X0, Y0) to (X2, Y2) and the PAT image data is acquired again. The changing of the start point is performed by, e.g., receiving an intervention by the user who has referenced the ultrasound image using an input unit.

By the changing of the start point of the ROI, in the C-mode image in FIG. 9B, a new ROI region 92 is set and a stripe 93 also becomes a measurement target. That is, the number of the stripes for which functional distribution information is generated increases. Then, the probe 5 scans the stripe 93 again without changing the position in the Y-direction to acquire data. At this time, the PAT image data of the entire ROI region 92 that has been changed is simultaneously acquired.

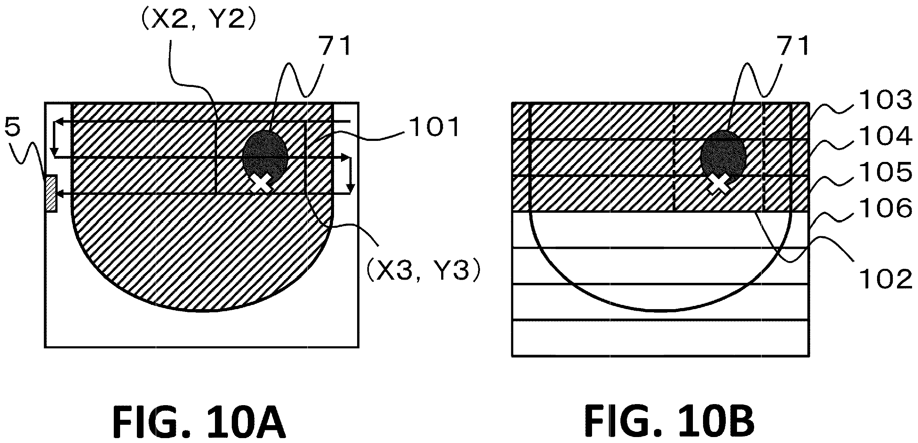

FIGS. 10A and 10B show the state after the set range has been adjusted with respect to the end point of the ROI. FIG. 10A shows a camera image. FIG. 10B shows a C-mode image. A range 101 shows the set range after ROI adjustment in the camera image (FIG. 10A) . By the setting, the end point of the ROI has been changed from (X1, Y1) to (X3, Y3).

As a result of the changing of the end point, ROI range setting 102 in a stripe 106 is no longer necessary in the C-mode image (FIG. 10B). That is, the stripes for which photoacoustic data eventually needs to be acquired are only stripes 103, 104, and 105. Consequently, the measurement time is reduced.

In the present embodiment, the shape of the lesion is recognized in the C-mode image. However, when a lesion is not found, the present invention is also applicable to a method in which the setting of the ROI range is cancelled to cancel the PAT image reconstruction.

In accordance with the present invention, as shown in the above comparative example, in the system which acquires the ultrasound image of the entire region of the object in real time and acquires the PAT image of the region of interest (ROI), the ROI adjustment can easily be performed even during scanning. Specifically, it becomes possible to adjust the ROI measurement conditions set in advance, while referencing an ultrasound image in real time during probe scanning.

As the measurement conditions to be adjusted here, not only the set range (size and position), but also the number of integrations for reducing noise, a gain (TGC) for adjusting a signal intensity, and the like can also be changed.

Note that, in each of the embodiment and example described above, it is possible to sequentially display ultrasound image data, while moving the probe in the X- and Y- directions relative to the object to scan the object. Therefore, the present invention is also applicable to an apparatus which images object interior information only by transmitting/receiving an ultrasound wave without detecting a photoacoustic wave.

While the present invention has been described with reference to exemplary embodiments, it is to be understood that the invention is not limited thereto, but is defined according to the scope of the accompanying claims.

**Claims**

1. An object information acquiring apparatus, comprising:

   a receiver (**14**) configured to receive an ultrasound wave transmitted to an object (**21**) and then reflected by the object and a photoacoustic wave generated in the object in response to an illumination with light, wherein the receiver (**14**) integrally includes a probe (**5**) that receives the ultrasound wave and a probe (**6**) that receives the photoacoustic wave;
   a scanner (**12**) configured to mechanically move the receiver (**14**) relative to the object along a path to scan the object;
   a processor (**9**) configured to generate structural distribution information on the interior of the object using the ultrasound wave and generate functional distribution information on the interior of the object using the photoacoustic wave;
   a display (11);
   a controller (**10**) configured to perform a control operation of causing the display (**11**) to display the structural distribution information and the functional distribution information; and
an input unit configured to receive a designation of a region of interest in the object,
**characterized in that**
the controller (**10**) performs a control operation of causing, during a period in which the receiver receives the photoacoustic wave from a partial region formed of at least a part of the object and in which the processor (**9**) generates the functional distribution information on the partial region, the display to sequentially display the structural distribution information generated by the processor (**9**) on the basis of the ultrasound wave reflected from the partial region of the object as the scanner (**12**) moves the receiver (**14**) to implement scanning in the partial region,
the processor (**9**) generates the functional distribution information using the photoacoustic wave in the region of interest,
the input unit is capable of receiving a change of the region of interest while the controller (**10**) causes the display (**11**) to sequentially display the structural distribution information, and
the scanner (**12**) changes the path of the receiver (**14**) on the basis of the changed region of interest.

2. The object information acquiring apparatus according to claim 1, further comprising:

   an imaging unit that captures an image of the object (**21**), wherein
   the controller is further configured to cause the display (**11**) to display the image obtained by the imaging unit together with the structural distribution information.

3. The object information acquiring apparatus according to any one of claim 1 or 2, wherein
   the controller is further configured to set region of interest on the basis of a marking formed on a surface of the object (**21**).

4. The object information acquiring apparatus according to any one of claims 1 to 3, wherein
   the scanner (**12**) moves the receiver (**14**) to implement scanning in a main scanning direction thereby forming stripes, and moves the receiver (**14**) to implement scanning in a subordinate scanning direction thereby moving the receiver (**14**) between the stripes.

5. The object information acquiring apparatus according to any one of claims 1 to 4, further comprising:

   a plate configured to hold the object, wherein
   the scanner (**12**) mechanically moves the receiver (**14**) over the plate to implement scanning.

6. The object information acquiring apparatus according to claim 5, wherein the processor (**9**) generates, as the structural distribution information, a C-mode image along the plate on a per-depth basis.

7. The object information acquiring apparatus according to claim 5, wherein the processor (**9**) generates, as the structural distribution information, a B-mode image on the basis of each position of scanning by the receiver (**14**).

8. The object information acquiring apparatus according to any one of claims 1 to 7, wherein the receiver (**14**) uses a common element for receiving the ultrasound wave and receiving the photoacoustic wave.

9. A method for controlling an object information acquiring apparatus including a receiver (**14**), wherein the receiver (**14**) integrally includes a probe (**5**) that receives an ultrasound wave and a probe (**6**) that receives a photoacoustic wave, a scanner (**12**) that mechanically moves the receiver relative to an object (**21**) along a path to scan the object, a processor (**9**), a display (**11**), a controller (**10**) that performs a control operation of causing the display (**11**) to display structural distribution information and functional distribution information, and an input unit that receives a designation of a region of interest in the object,
   the method comprising:

   operating the receiver (**14**) to receive an ultrasound wave transmitted to the object and then reflected by the object;
   operating the receiver (**14**) to receive a photoacoustic wave generated in the object in response to an illumination with light;
   operating the processor (**9**) to generate structural distribution information on the interior of the object using the ultrasound wave; and
   operating the processor (**9**) to generate functional distribution information on the interior of the object using the photoacoustic wave,

**characterized by**

operating the controller (**10**) to perform a control operation of causing, during a period in which the receiver (**14**) receives the photoacoustic wave from a partial region formed of at least a part of the object and in which the processor (**9**) generates the functional distribution information on the partial region, the display (**11**) to sequentially display the structural distribution information generated by the processor (**9**) on the basis of the ultrasound wave reflected from the partial region of the object as the scanner (**12**) moves the receiver to implement scanning in the partial region,

operating the processor (**9**) to generate the functional distribution information using the photoacoustic wave in the region of interest,

operating the input unit to receive a change of the region of interest while the controller (**10**) causes the display (**11**) to sequentially display the structural distribution information, and

operating the scanner (**12**) to change the path of the receiver (**14**) on the basis of the changed region of interest.

10. The method for controlling the object information acquiring apparatus according to claim 9, the object information acquiring apparatus further including an imaging unit, the method further comprising:

operating the imaging unit to capture an image of the object, and

operating the controller to cause the display to display the image obtained by the imaging unit together with the structural distribution information.

11. The method for controlling the object information acquiring apparatus according to claim 9 or 10, further comprising:
operating the controller to set the region of interest on the basis of a marking formed on a surface of the object.

12. The method for controlling the object information acquiring apparatus according to any one of claims 9 to 11, further comprising:
operating the scanner to move the receiver to implement scanning in a main scanning direction thereby forming stripes, and moves the receiver to implement scanning in a subordinate scanning direction thereby moving the receiver between the stripes.

13. The method for controlling the object information acquiring apparatus according to any one of claims 9 to 12, the object information acquiring apparatus further including a plate, the method further comprising:

operating the plate to hold the object, and

operating the scanner to mechanically move the receiver over the plate to implement scanning.

14. The method for controlling the object information acquiring apparatus according to claim 13, further comprising:
operating the processor (9) to generate, as the structural distribution information, a C-mode image along the plate on a per-depth basis.

15. The method for controlling the object information acquiring apparatus according to claim 13, further comprising:
operating the processor (**9**) to generate, as the structural distribution information, a B-mode image on the basis of each position of scanning by the receiver (**14**).

16. The method for controlling the object information acquiring apparatus according to any one of claims 9 to 15, further comprising:
operating the receiver (**14**) to use a common element for receiving the ultrasound wave and receiving the photoacoustic wave.

**Patentansprüche**

1. Objektinformationenbezugsvorrichtung, mit:

einem Empfänger (**14**), der konfiguriert ist, um eine Ultraschallwelle, die zu einem Objekt (**21**) übertragen und dann von dem Objekt reflektiert wird, und eine photoakustische Welle, die in dem Objekt als Reaktion auf eine Beleuchtung durch Licht erzeugt wird, zu empfangen, wobei der Empfänger (**14**) integral eine Sonde (**5**), die die Ultraschallwelle empfängt, und eine Sonde (**6**), die die photoakustische Welle empfängt, umfasst;

einer Abtasteinrichtung (**12**), die konfiguriert ist, um den Empfänger (**14**) relativ zu dem Objekt mechanisch entlang eines Pfades zum Abtasten des Objekts zu bewegen;

einem Prozessor (**9**), der konfiguriert ist, um strukturelle Verteilungsinformationen über das Innere des Objekts unter Verwendung der Ultraschallwelle zu erzeugen, und funktionale Verteilungsinformationen über das Innere des Objekts unter Verwendung der photoakustischen Welle zu erzeugen;

einer Anzeige (**11**);

einer Steuerung (**10**), die konfiguriert ist, um einen Steuervorgang zum Bewirken der Anzeige (**11**), um die strukturellen Verteilungsinformationen und die funktionalen Verteilungsinformationen anzuzeigen; und

einer Eingabeeinheit, die konfiguriert ist, um eine Zuweisung eines Bereichs von Interesse in dem Objekt zu empfangen,

**dadurch gekennzeichnet, dass**

die Steuerung (**10**) einen Steuervorgang zum Bewirken der Anzeige, um während eines Zeitraums, in dem der Empfänger die photoakustische Welle aus einem Teilbereich empfängt, der aus mindestens einem Teil des Objekts besteht, und in dem der Prozessor (**9**) die funktionalen Verteilungsinformationen über den Teilbereich erzeugt, die vom Prozessor (**9**) erzeugten strukturelle Verteilungsinformationen basierend auf der Ultraschallwelle, die von dem Teilbereich des Objekts reflektiert wird, sequentiell anzuzeigen, wenn der Scanner (**12**) den Empfänger (**14**) bewegt, um eine Abtastung in dem Teilbereich durchzuführen,

der Prozessor (**9**) die funktionalen Verteilungsinformationen unter Verwendung der photoakustischen Welle in dem Bereich von Interesse erzeugt,

die Eingabeeinheit in der Lage ist, eine Änderung des Bereichs von Interesse zu empfangen, während die Steuerung (**10**) die Anzeige (**11**) bewirkt, die strukturellen Verteilungsinformationen sequentiell anzuzeigen, und die Abtasteinrichtung (**12**) den Pfad des Empfängers (**14**) basierend auf dem geänderten Bereich von Interesse ändert.

2.  Objektinformationenbezugsvorrichtung gemäß Anspruch 1, weiterhin mit:

einer Bildgebungseinheit, die ein Bild des Objekts (**21**) aufnimmt, wobei
die Steuereinheit ferner konfiguriert ist, um die Anzeige (**11**) zu bewirken, um das durch die Bildgebungseinheit erhaltene Bild zusammen mit den strukturelle Verteilungsinformationen anzuzeigen.

3.  Objektinformationenbezugsvorrichtung gemäß einem der Ansprüche 1 oder 2, wobei
die Steuereinheit ferner konfiguriert ist, um einen Bereich von Interesse basierend auf einer auf einer Oberfläche des Objekts (**21**) ausgebildeten Markierung einzustellen.

4.  Objektinformationenbezugsvorrichtung gemäß einem der Ansprüche 1 bis 3, wobei
die Abtasteinrichtung (**12**) den Empfänger (**14**) bewegt, um ein Abtasten in einer Hauptabtastrichtung zu implementieren, wodurch Bänder gebildet werden, und den Empfänger (**14**) bewegt, um das Abtasten in einer sekundären Abtastrichtung zu implementieren, wodurch der Empfänger (**14**) zwischen den Bändern bewegt wird.

5.  Objektinformationenbezugsvorrichtung gemäß einem der Ansprüche 1 bis 4, weiterhin mit:
einer Platte, die konfiguriert ist, um das Objekt zu halten, wobei die Abtasteinrichtung (**12**) den Empfänger (**14**) mechanisch über die Platte bewegt, um eine Abtastung zu implementieren.

6.  Objektinformationenbezugsvorrichtung gemäß Anspruch 5, wobei der Prozessor (**9**) als die strukturellen Verteilungsinformationen ein C-Modus-Bild entlang der Platte auf Grundlage pro Tiefe erzeugt.

7.  Objektinformationenbezugsvorrichtung gemäß Anspruch 5, wobei der Prozessor (**9**) als die strukturellen Verteilungsinformationen ein B-Modus-Bild auf Grundlage jeder Position der vom Empfänger (**14**) durchgeführten Abtastung erzeugt.

8.  Objektinformationenbezugsvorrichtung gemäß einem der Ansprüche 1 bis 7, wobei der Empfänger (**14**) ein gemeinsames Element zum Empfangen der Ultraschallwelle und zum Empfangen der photoakustischen Welle verwendet.

9.  Verfahren zum Steuern einer Objektinformationserfassungsvorrichtung mit einem Empfänger (**14**), wobei der Empfänger (**14**) integral eine Sonde (**5**), die eine Ultraschallwelle empfängt, und eine Sonde (**6**), die eine photoakustische Welle empfängt, umfasst, einer Abtasteinrichtung (**12**), die den Empfänger (**14**) relativ zu einem Objekt (**21**) mechanisch entlang eines Pfades zum Abtasten des Objekts bewegt, einem Prozessor (**9**), einer Anzeige (**11**), einer Steuerung (**10**), die einen Steuervorgang zum Bewirken der Anzeige (**11**), um strukturelle Verteilungsinformationen und funktionale Verteilungsinformationen anzuzeigen, durchführt, und einer Eingabeeinheit, die eine Zuweisung eines Bereichs von Interesse in dem Objekt empfänmgt,
wobei das Verfahren aufweist:

Betreiben des Empfängers (**14**) zum Empfangen einer Ultraschallwelle, die auf das Objekt übertragen und dann von dem Objekt reflektiert wird;
Betreiben des Empfängers (**14**) zum Empfangen einer photoakustischen Welle, die in dem Objekt als Reaktion auf die Beleuchtung durch Licht erzeugt wird;
Betreiben des Prozessors (**9**), um strukturelle Verteilungsinformationen über das Innere des Objekts unter Verwendung der Ultraschallwelle zu erzeugen;
Betreiben des Prozessors (**9**), um funktionale Verteilungsinformationen über das Innere des Objekts unter Verwendung der photoakustischen Welle zu erzeugen;
**gekennzeichnet durch**
Betreiben der Steuerung (**10**), um einen Steuervorgang durchzuführen, der darin besteht, während eines Zeitraums, in dem der Empfänger (**14**) die photoakustische Welle aus einem Teilbereich empfängt, der aus mindestens einem Teil des Objekts gebildet ist und in dem der Prozessor (**9**) die funktionale Verteilungsinformation über den Teilbereich erzeugt, die Anzeige (**11**) die vom Prozessor (**9**) erzeugten strukturelle Verteilungsinformationen basierend auf der Ultraschallwelle, die von dem Teilbereich des Objekts reflektiert wird, sequentiell anzeigt, wenn der Scanner (**12**) den Empfänger bewegt, um eine Abtastung in dem Teilbereich durchzuführen,
Betreiben des Prozessors (**9**), um funktionale Verteilungsinformationen unter Verwendung der photoakustischen Welle in dem Bereich von Interesse zu erzeugen,
Betreiben der Eingabeeinheit, um eine Änderung des Bereichs von Interesse zu empfangen, während die Steuerung (**10**) die Anzeige (**11**) bewirkt, um die strukturellen Verteilungsinformationen sequentiell anzuzeigen, und
Betreiben der Abtasteinrichtung (**12**), um den Pfad des Empfängers (**14**) basierend auf dem geänderten Bereich von Interesse zu ändern.

10. Verfahren zum Steuern der Objektinformationenbezugsvorrichtung gemäß Anspruch 9, wobei die Objektinformationenbezugsvorrichtung ferner eine Bildgebungseinheit umfasst, und das Verfahren weiterhin aufweist:

Betreiben der Bildeinheit, um ein Bild des Objekts aufzunehmen, und
Betreiben der Steuereinheit, um die Anzeige zu veranlassen, das von der Bildeinheit erhaltene Bild in Verbindung mit den strukturellen Verteilungsinformationen anzuzeigen.

11. Verfahren zum Steuern der Objektinformationenbezugsvorrichtung gemäß Anspruch 9 oder 10, weiterhin mit:
Betreiben der Steuereinheit, um einen Bereich von Interesse basierend auf einer auf einer Oberfläche des Objekts (**21**) ausgebildeten Markierung einzustellen.

12. Verfahren zum Steuern der Objektinformationenbezugsvorrichtung gemäß einem der Ansprüche 9 bis 11, weiterhin mit:
Betreiben der Abtasteinrichtung, um den Empfänger zu bewegen, um das Abtasten in einer Hauptabtastrichtung zu implementieren, wodurch Bänder gebildet werden, und um den Empfänger zu bewegen, um das Abtasten in einer sekundären Abtastrichtung zu implementieren, wodurch der Empfänger zwischen den Bändern bewegt wird.

13. Verfahren zum Steuern der Objektinformationenbezugsvorrichtung gemäß einem der Ansprüche 9 bis 12, wobei die Objektinformationenbezugsvorrichtung ferner eine Platte umfasst, und das Verfahren weiterhin aufweist:

Betreiben der Platte, um das Objekt zu halten, und
Betreiben der Abtasteinrichtung, um den Empfänger mechanisch über die Platte zu bewegen, um ein Abtasten zu implementieren.

14. Verfahren zum Steuern der Objektinformationenbezugsvorrichtung gemäß Anspruch 13, weiterhin mit:
Betreiben des Prozessors (**9**), um, als die strukturellen Verteilungsinformationen, ein C-Modus-Bild entlang der Platte auf Grundlage pro Tiefe zu erzeugen.

15. Verfahren zum Steuern der Objektinformationenbezugsvorrichtung gemäß Anspruch 13, weiterhin mit:
Betreiben des Prozessors (**9**), um, als die strukturellen Verteilungsinformationen, ein B-Modus-Bild auf Grundlage jeder Position der vom Empfänger (**14**) durchgeführten Abtastung zu erzeugen.

**16.** Verfahren zum Steuern der Objektinformationenbezugsvorrichtung gemäß einem der Ansprüche 9 bis 15, weiterhin mit:

Betreiben des Empfängers (**14**), um ein gemeinsames Element zum Empfangen der Ultraschallwelle und zum Empfangen der photoakustischen Welle zu verwenden.

**Revendications**

**1.** Appareil d'acquisition d'informations d'objet, comprenant :

un récepteur (14) configuré pour recevoir une onde ultrasonore transmise vers un objet (21) puis réfléchie par l'objet et une onde photoacoustique générée dans l'objet en réponse à une illumination par de la lumière, où le récepteur (14) comporte de manière intégrale une sonde (5) qui reçoit l'onde ultrasonore et une sonde (6) qui reçoit l'onde photoacoustique ;
un dispositif de balayage (12) configuré pour déplacer mécaniquement le récepteur (14) par rapport à l'objet le long d'un trajet afin de balayer l'objet ;
un processeur (9) configuré pour générer des informations de distribution structurelle concernant l'intérieur de l'objet en utilisant l'onde ultrasonore et pour générer des informations de distribution fonctionnelle concernant l'intérieur de l'objet en utilisant l'onde photoacoustique ;
un afficheur (11) ;
une unité de commande (10), configurée pour effectuer une opération de commande consistant à amener l'afficheur (11) à afficher les informations de distribution structurelle et les informations de distribution fonctionnelle ; et
une unité d'entrée configurée pour recevoir une désignation d'une région d'intérêt dans l'objet,
**caractérisé en ce que**
l'unité de commande (10) effectue une opération de commande consistant à amener, pendant une période au cours de laquelle le récepteur reçoit l'onde photoacoustique en provenance d'une région partielle formée d'au moins une partie de l'objet et au cours de laquelle le processeur (9) génère des informations de distribution fonctionnelle concernant la région partielle, l'afficheur à afficher séquentiellement les informations de distribution structurelle générées par le processeur (9) sur la base de l'onde ultrasonore réfléchie par la région partielle de l'objet lorsque le dispositif de balayage (12) déplace le récepteur (14) afin de mettre en œuvre un balayage dans la région partielle,
le processeur (9) génère les informations de distribution fonctionnelle en utilisant l'onde photoacoustique dans la région d'intérêt,
l'unité d'entrée est capable de recevoir une modification de la région d'intérêt pendant que l'unité de commande (10) amène l'afficheur (11) à afficher séquentiellement les informations de distribution structurelle, et
le dispositif de balayage (12) modifie le trajet du récepteur (14) sur la base de la région d'intérêt modifiée.

**2.** Appareil d'acquisition d'informations d'objet selon la revendication 1, comprenant en outre :

une unité de formation d'image qui acquiert une image de l'objet (21), où
l'unité de commande est en outre configurée pour amener l'afficheur (11) à afficher l'image obtenue par l'unité de formation d'image en association avec les informations de distribution structurelle.

**3.** Appareil d'acquisition d'informations d'objet selon l'une quelconque des revendications 1 ou 2, dans lequel l'unité de commande est en outre configurée pour définir une région d'intérêt sur la base d'un marquage formé sur une surface de l'objet (21).

**4.** Appareil d'acquisition d'informations d'objet selon l'une quelconque des revendications 1 à 3, dans lequel le dispositif de balayage (12) déplace le récepteur (14) afin de mettre en œuvre un balayage dans une direction de balayage principale pour ainsi former des bandes, et déplace le récepteur (14) afin de mettre en œuvre un balayage dans une direction de balayage secondaire pour ainsi déplacer le récepteur (14) entre les bandes.

**5.** Appareil d'acquisition d'informations d'objet selon l'une quelconque des revendications 1 à 4, comprenant en outre :

une plaque configurée pour maintenir l'objet, où
le dispositif de balayage (12) déplace mécaniquement le récepteur (14) au-dessus de la plaque afin de mettre en œuvre un balayage.

**6.** Appareil d'acquisition d'informations d'objet selon la revendication 5, dans lequel le processeur (9) génère, en tant qu'informations de distribution structurelle, une image en mode C le long de la plaque en fonction de la profondeur.

**7.** Appareil d'acquisition d'informations d'objet selon la revendication 5, dans lequel le processeur (9) génère, en tant qu'informations de distribution structurelle, une image en mode B sur la base de chaque position du balayage effectué par le récepteur (14).

**8.** Appareil d'acquisition d'informations d'objet selon l'une quelconque des revendications 1 à 7, dans lequel le récepteur (14) utilise un élément commun pour recevoir l'onde ultrasonore et recevoir l'onde photoacoustique.

**9.** Procédé de commande d'un appareil d'acquisition d'informations d'objet comportant un récepteur (14), dans lequel le récepteur (14) comporte de manière intégrale une sonde (5) qui reçoit une onde ultrasonore et une sonde (6) qui reçoit une onde photoacoustique, un dispositif de balayage (12) qui déplace mécaniquement le récepteur (14) par rapport à un objet (21) le long d'un trajet afin de balayer l'objet, un processeur (9), un afficheur (11), une unité de commande (10) qui effectue une opération de commande consistant à amener l'afficheur (11) à afficher des informations de distribution structurelle et des informations de distribution fonctionnelle, et une unité d'entrée qui reçoit une désignation d'une région d'intérêt dans l'objet,
le procédé comprenant les étapes consistant à :

faire fonctionner le récepteur (14) afin de recevoir une onde ultrasonore transmise vers l'objet puis réfléchie par l'objet ;
faire fonctionner le récepteur (14) pour recevoir une onde photoacoustique générée dans l'objet en réponse à une illumination par de la lumière ;
faire fonctionner le processeur (9) pour générer des informations de distribution structurelle concernant l'intérieur de l'objet en utilisant l'onde ultrasonore ;
faire fonctionner le processeur (9) pour générer des informations de distribution fonctionnelle concernant l'intérieur de l'objet en utilisant l'onde photoacoustique ;
**caractérisé par** les étapes consistant à
faire fonctionner l'unité de commande (10) pour effectuer une opération de commande consistant à amener, pendant une période au cours de laquelle le récepteur (14) reçoit l'onde photoacoustique en provenance d'une région partielle formée d'au moins une partie de l'objet et au cours de laquelle le processeur (9) génère les informations de distribution fonctionnelle concernant la région partielle, l'afficheur (11) à afficher séquentiellement les informations de distribution structurelle générées par le processeur (9) sur la base de l'onde ultrasonore réfléchie par la région partielle de l'objet lorsque le dispositif de balayage (12) déplace le récepteur pour mettre en œuvre un balayage dans la région partielle,
faire fonctionner le processeur (9) pour générer les informations de distribution fonctionnelle en utilisant l'onde photoacoustique dans la région d'intérêt,
faire fonctionner l'unité d'entrée pour recevoir une modification de la région d'intérêt pendant que l'unité de commande (10) amène l'afficheur (11) à afficher séquentiellement les informations de distribution structurelle, et
faire fonctionner le dispositif de balayage (12) pour modifier le trajet du récepteur (14) sur la base de la région d'intérêt modifiée.

**10.** Procédé de commande de l'appareil d'acquisition d'informations d'objet selon la revendication 9, l'appareil d'acquisition d'information d'objet comportant en outre une unité de formation d'image, le procédé comprenant en outre les étapes consistant à :

faire fonctionner l'unité de formation d'image pour acquérir une image de l'objet, et
faire fonctionner l'unité de commande pour amener l'afficheur à afficher l'image obtenue par l'unité de formation d'image en association avec les informations de distribution structurelle.

**11.** Procédé de commande de l'appareil d'acquisition d'informations d'objet selon la revendication 9 ou 10, comprenant en outre l'étape consistant à :
faire fonctionner l'unité de commande pour définir la région d'intérêt sur la base d'un marquage formé sur une surface de l'objet.

**12.** Procédé de commande de l'appareil d'acquisition d'informations d'objet selon l'une quelconque des revendications 9 à 11, comprenant en outre l'étape consistant à :
faire fonctionner le dispositif de balayage pour déplacer le récepteur afin de mettre en œuvre un balayage dans une direction de balayage principale pour ainsi former des bandes, et déplacer le récepteur afin de mettre en œuvre un balayage dans une direction de balayage secondaire pour ainsi déplacer le récepteur entre les bandes.

13. Procédé de commande de l'appareil d'acquisition d'informations d'objet selon l'une quelconque des revendications 9 à 12, l'appareil d'acquisition d'informations d'objet comportant en outre une plaque, le procédé comprenant en outre les étapes consistant à :

   faire fonctionner la plaque pour maintenir l'objet, et
   faire fonctionner le dispositif de balayage pour déplacer mécaniquement le récepteur au-dessus de la plaque afin de mettre en œuvre un balayage.

14. Procédé de commande de l'appareil d'acquisition d'informations d'objet selon la revendication 13, comprenant en outre l'étape consistant à :
   faire fonctionner le processeur (9) pour générer, en tant qu'informations de distribution structurelle, une image en mode C le long de la plaque en fonction de la profondeur.

15. Procédé de commande de l'appareil d'acquisition d'informations d'objet selon la revendication 13, comprenant en outre l'étape consistant à :
   faire fonctionner le processeur (9) pour générer, en tant qu'informations de distribution structurelle, une image en mode B sur la base de chaque position du balayage effectué par le récepteur (14).

16. Procédé de commande de l'appareil d'acquisition d'informations d'objet selon l'une quelconque des revendications 9 à 15, comprenant en outre l'étape consistant à :
   faire fonctionner le récepteur (14) pour utiliser un élément commun destiné à recevoir l'onde ultrasonore et à recevoir l'onde photoacoustique.

FIG. 1

**FIG. 2**

**FIG. 3**

**FIG. 4**

FIG. 5A

FIG. 5B

FIG. 5C

**FIG. 6A**

(1) Z＝10mm      (2) Z＝15mm      (3) Z＝20mm

**FIG. 6B**

# FIG. 7A

# FIG. 7B

**FIG. 8A**

**FIG. 8B**

**FIG. 9A**

**FIG. 9B**

EP 2 853 917 B1

**FIG. 10A**

**FIG. 10B**

EP 2 853 917 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010269046 A
- WO 2011074656 A1
- EP 1561424 A1
- WO 2013065249 A1

**Non-patent literature cited in the description**

- **SRIRANG MANOHAR et al.** The Twente photoacoustic mammoscope: system overview and performance. *Physics in Medicine and Biology,* 2005, vol. 50, 2543-2557